# EUROPEAN PATENT SPECIFICATION

(11) **EP 2 420 187 B1**
(45) Date of publication and mention of the grant of the patent: **13.02.2013**
(21) Application number: 10172963.0
(22) Date of filing: 16.08.2010
(51) Int. Cl.: A61B 5/04, B25J 7/00, A61B 19/00

(54) **Individually adjustable multi-channel systems in vivo recording**
Individuell einstellbare Mehrkanalsysteme bei der In-Vivo-Aufzeichnung
Enregistrement in vivo de systèmes multicanaux ajustables individuellement

(43) Date of publication of application: 22.02.2012
(73) Proprietor: Precision Bioinstrument LLC, Westfield, NJ 07090 (US)
(72) Inventor: Bai, Yun, Westfield NJ New Jersey 07090 (US)
(74) Representative: Grund, Martin

(56) References cited:
- EP-A2- 0 574 022
- WO-A1-2009/154308
- US-A- 4 894 579
- US-A- 5 928 143
- US-A1- 2003 065 250

## Description

### FIELD OF INVENTION

The present application is in the field of medical device. In one embodiment, the device is a biological instrument used in electrophysiological recording. In particular, the device is an individually adjustable multi-channel system used for recording electric activities of cells.

### BACKGROUND OF INVENTION

Multi-unit recording techniques have great potential for exploring how neurons process information, how signals from individual neurons are computed to generate a circuitry level output, and how neuronal activities are translated into behavioral outputs. Significant discoveries in neural biology have been made by using these techniques. For instance, Koch et al first convincingly demonstrated that even a simple behavioral output is mediated by a large number of neurons from different brain areas (Koch C and Davis JL, Large-scale neuronal theories of the brain; pp343 Cambridge, MA Bradford Book (1994)). Aertsen et al also demonstrated that the temporal pattern of neuronal spikes encodes the functional output of cortex (Science 278:1950-3. (1997)). These ground-breaking discoveries have not only revolutionized the view on neuronal activities, but also demonstrated that the multi-channel recording is a powerful tool in biomedical research.

In contrast to its great potential, multi-unit recording techniques have not been widely used thus far due to a technical drawback associated with the currently available multi-channel systems. A standard multi-channel setup currently available in the art, such as the products provided by Triangle BioSystems (NC) and Multichannel Systems (Germany), usually comprise of four key components: i) multi-electrode set that usually contains four to hundreds of individual microelectrodes to acquire signals from individual neurons; ii) pre-amplifier that keeps the fidelity of the original signal; iii) main amplifier that amplifies the targeted signals; and iv) software that commands the recording. The multi-electrode set has not been designed in a way to meet the experimental needs of current electrophysiology studies. One fundamental flaw of the design is that the electrodes in the multi-electrode sets are all fixed and not adjustable in an array. The multi-unit recording should allow simultaneous recording of signals from individual neurons in a real time manner in free-moving animals. However, the fixed electrodes within the array, once implanted in the brain of a tested animal, usually cannot accomplish their full capacity. An electrode cannot detect any meaningful signal unless there is a neuron by chance located adjacent to it. Thus, although the multi-electrode set comprises a large number of electrodes, the number of electrodes that can practically acquire valid signals is relatively small, for example, less than 10-15% of the total electrodes. As a result, the current commercially available multi-unit recording systems are very inefficient despite the high demand for these techniques by neuroscience research laboratories. Therefore, it is of significant need to develop electrodes whose position can be individually adjusted during the electrophysiological recording. It is also of great need to develop micro-driving apparatus that can control the movement of electrodes in a micro scale.

Bossi *et al.* discloses a uni-directional microactuation device for improving performance of intraneural longitudinal interfaces with the peripheral nervous system using "serpentine like" shape memory thin films. (IEEE RAS and EMBS International Conference on Biomedical Robotics and Biomechatrons (2006); IEEE Transactions on Biomedical Engineering, Vol. 54, No. 6, p.p.1115-1119 (2007)).

US 2003/0065250 A1 discloses a self-propelled endoscope device that is capable of peristaltic locomotion and comprises actuators with shape memory alloy (SMA) springs to bend the actuators by application of an electric current.

EP 0 574 022 A2 discloses a movement actuator including an elongate fiber that carries SMA strips on its surface for effecting movement of the actuator.

US 5,928,143 discloses an electrode array for use in neurophysiological research on freely moving animals comprising a plurality of independently movable electrodes.

US 4,894,579 discloses an apparatus for effecting a fine movement of an object comprising a moving and an inertia member connected by a piezoelectric/electrostrictive element capable of generating impact force.

The inventors of the present application have developed a micro-driving apparatus comprising a shape memory alloy (SMA) wire together with a mechanism to make the SMA change its length in the desired manner, and thereby adjust the position of the recording element of each electrode. The present driving apparatus can be made very small because shape memory wires can generate enough force with very small diameter, which is less than a couple of hundred microns. Using this micro-driving apparatus, the inventors of the present application further developed a novel, adjustable multi-electrode system (AMES), which comprises hundreds and thousands of single electrodes, each of which can be elongated or withdrawn to an optimal position in acquiring neuronal signals in awake, freely moving animals. The present AMES enables every recording element within the system to be positioned adjacent to a neuron to acquire a valid signal. Thus, the present AMES technique is highly productive and user-friendly.

### SUMMARY OF INVENTION

In one embodiment, the present invention includes a micro-driving apparatus comprising a shape memory alloy wire coupled to an electrode comprising a lumen, an elongated body, a proximal end and a distal end.

The shape memory alloy wire (SMA) is coupled to the proximal end of the electrode.

In one aspect, the SMA wire is straight.

In another aspect, the SMA wire is parallely coupled with the electrode.

The micro-driving apparatus further comprises a commanding circuit connected to the wire for providing electrical current to the wire, wherein the length of the wire will be retracted when a commanding current is applied to the circuit.

In a further aspect, the commanding current is a direct circuit (DC) current or a pulse width module (PWM) current.

In yet a further aspect, the retraction of the wire results in retraction of said electrode.

The wire is stretched with a biasing element, and wherein said retracted wire is restored to its original shape by the biasing element.

In a further aspect, the biasing element is a spring.

In another aspect, the wire has a diameter of less than 50 µm.

In another aspect, the wire has a length of at least 1mm.

In another aspect, the present apparatus has a diameter of less than 500 µm.

In another aspect, the wire is a nitinol wire.

In another example, the apparatus comprises a bi-directional mechanism capable of moving the electrode in two opposite directions, wherein said mechanism comprises two SMW wires, each of which is connected independently to a corresponding commanding circuit.

In yet another example, the apparatus comprises a bi-directional mechanism capable of moving the electrode in two opposite directions, wherein said mechanism comprises a single SMW wire with one part of the SMW wire connected to a commanding circuit while the other part of the SMW wire connected to another commanding circuit.

In another example, the apparatus includes a driving device comprising a first micro-driving apparatus comprising a first shape memory alloy wire connected to a first commanding circuit; and a second micro-driving apparatus comprising a second shape memory alloy wire connected to a second commanding circuit, wherein said second wire and said first wire are in parallel position and are connected by a bridging device, and wherein said first wire is connected to an electrode comprising a lumen, an elongated body, a proximal end and a distal end, wherein said wire is coupled to the proximal end of the electrode.

In one aspect, the length of the first wire will change when a commanding current is applied to the first circuit, and said change results in the movement of the electrode.

In another aspect, the length of the second wire will change when a commanding current is applied to the second circuit, and said change results in the movement of the electrode in a direction opposite to the movement caused by the first wire.

The present invention includes an adjustable electrode apparatus comprising: 1) an electrode comprising a lumen, an elongated body, a proximal end and a distal end, 2) a shape memory alloy wire coupled to the proximal end of the microelectrode for extending and retracting the microelectrode, wherein the alloy wire is stretched with a biasing element; and 3) a commanding circuit connected to the alloy wire for providing an electrical current to the wire.

The apparatus further comprises a locking device to lock the electrode in a desired position, wherein said locking device locks an electrode in a desired position via a spring load horizontally wrapping the electrode.

In another aspect, a curved SMA wire is stretched by a spring positioned horizontally with said curved wire, wherein said curved wire connected to an electric circuit, wherein the curved wire will be retracted when a commanding current is applied to the circuit.

In yet another aspect, the retraction results in loosening the lock of the electrode by said locking device so that the position of the electrode can be readjusted.

In one aspect, the electrode can be elongated up to about 400 microns or up to twenty neurons.

In another aspect, the electrode is connected to a preamplifier, which is further connected to a data processing system.

In another aspect, the electrode comprises electrode tubing and insulator.

In one aspect, the electrode tubing has a tungsten wire sitting throughout the inside of the tubing or is filled with electrolyte with the end sealed with conductive epoxy or glue.

In one aspect, the electrode tubing is within a guide tubing with one end coming out of the guide tubing and acting as recording end and the other end acts as a supporting end.

In another aspect, the driving apparatus of the present application is connected proximally to the supporting end of guide tubing.

In yet another aspect, the apparatus includes an adjustable multi-electrode system comprising at least one array of electrodes of the present application, wherein the position of each electrode of said system is individually adjustable.

In one aspect, the electrodes measure neuronal signal of a wake animal. In a preferred aspect, the animal is a freely moving animal.

In another aspect, each electrode of the multi-electrode system is positioned adjacent to a neuron.

In another aspect, the multi-electrode system comprises more than 50 electrodes in an area of 1x1 cm².

In another aspect, the shape or contour of the multi-electrode system is adjustable.

In another aspect, the array of the electrodes in the multi-electrode system is organized in a way that each electrode radiates to a spherical surrounding with different lengths.

In another aspect, some electrodes of the multi-electrode system said system is capable of delivering electric current or voltage stimulus to a neuron or a subset group of neurons of said nuclei.

In a further aspect, other electrodes of the multi-electrode system record the signals from said stimulated neuron or stimulated subset of neurons.

In yet another aspect, said recording occurs concurrently or subsequently with said stimulation.

Disclosed is a method of adjusting the position of recording element of an electrode comprising connecting the electrode with the apparatus of and retracting or elongating the electrode with the apparatus.

Disclosed is a method of recording neuronal activity of a group of neuron of a subject in vivo comprising 1) install an AMES of the present application in the brain of said subject and 2) adjusting positions of each electrode of said system so that each electrode is in an optimal position for recording activities of each neuron of said group of neurons; and 3) recording the activities of each neurons of said group simultaneously.

In one aspect, said group of neuron is a nucleus.

In another aspect, said subject is a rodent,

In yet another aspect, said subject moves freely during the recording.

Disclosed is a method of controlling the movement of an electrode comprising: 1) connecting a micro-driving apparatus comprising a shape memory alloy wire or a wire having similar length-changing characteristics as a shape memory wire; and 2) retracting or elongating the electrode by adjusting the length of said wire.

### BRIEF DESCRIPTION OF DRAWINGS

Figure 1 is a diagram of the electrode apparatus of the present application.
Figure 2A is a diagram of the electrode apparatus comprising the dual SMA wire bi-directional driving apparatus of the present application.
Figure 2B is a diagram of the electrode apparatus comprising the dual SMA driving apparatus and a locking device of the present application.
Figure 2C is a diagram of the electrode apparatus comprising the single SMA bi-directional driving apparatus.
Figure 3 is a diagram of the dual driving device of the present application.

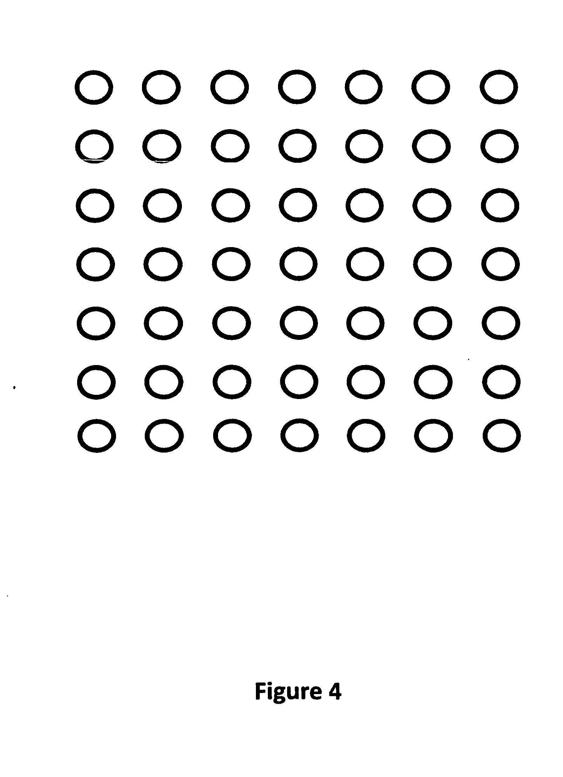

Figure 4 is a diagram showing the top view of an exemplary recording array of AMES.

### DETAILED DESCRIPTION OF INVENTIONS

### Definition:

The term "driving apparatus" refers to a device that is capable of controlling the movement of the recording element (or recording tip) of an electrode inside a tissue or a cell, preferably neuronal cells, for example, nuclei, ganglion, or single neuron. A "micro-driving apparatus" refers to a driving apparatus of small dimensions, such as having a diameter less than 10, 5, 2, 1mm, or less than 500, 300, 200, 100, or 50 µm.

The term "shape memory alloy" used herein refers to is an alloy that "remembers" its original, cold, forged shape, and which returns to that shape after being deformed by applying heat. Exemplary "shape memory alloy" includes the copper-zinc-aluminum-nickel, copper-aluminum-nickel and nickel-titanium (NiTi) alloys. Other SMA's can also be prepared by alloying zinc, copper, gold, and iron. In a preferred embodiment, the shape memory alloy is nitinol.

A wire has similar length-changing characteristics as a shape memory wire when it "remembers" its original, cold, forged shape, and which returns to that shape after being deformed by applying heat. The term "commanding current" refers to an electrical current that is applied to a circuit comprising a shape memory alloy wire. The current generates sufficient heat in the circuit so that the shape memory alloy wire changes its shape due to the heat.

The term "commanding circuit" refers to an electrical circuit that delivers a commanding current to the shape memory alloy wire.

The term "direct current" refers to a current with the unidirectional flow of electric charge. Direct current is produced by such sources as batteries, thermocouples, solar cells, and commutator-type electric machines of the dynamo type. Direct current may flow in a conductor such as a wire, but can also flow through semiconductors, insulators, or even through a vacuum, as in electron or ion beams. The electric current flows in a constant direction, distinguishing it from alternating current (AC), which is the movement (or flow) of electric charge periodically reverses direction.

The term "pulse width module (PWM) current" refers to an electric current generated by a PWM power source, providing intermediate amounts of electrical power between fully on and fully off. A PWM variable-power scheme switches the power quickly between fully on and fully off at the switching rate much faster than what would affect the load, which is to say the device that uses the power. Basically, a PWM variable-power scheme switches the power quickly between fully on and fully off -- e.g. several times a minute in an electric stove, 120 Hz in a lamp dimmer, and well into the tens or hundreds of kHz in a computer power supply (which has a regulated output). In any event, the switching rate is much faster than what would affect the load, which is to say the device that uses the power. In practice, applying full power for part of the time does not cause any problems. In an exemplary embodiment of the present application, the switch rate of the PWM power has a range of 0-500 MHz, 1-1000 Hz, 10-500 HZ, or 10-100 Hz.

The term "electrode" refers to a device that is capable of recording electrical properties of biological cells and tissues. The electrode may be used for the measurements of voltage change or electric current on a wide variety of scales from single ion channel proteins to whole organs like the heart or the brain. In one aspect, the electrode may be used for the measurements of the electrical activity of neurons, and particularly action potential activity. In an exemplary embodiment, an electrode comprises a lumen, an elongated body, a proximal end and a distal end, with the distal end as the recording element (recoding tip) for recoding the electric activity of a cell or a tissue and the proximal end as the supporting element (supporting tip) to anchor the electrode to a desired position. The driving apparatus of the present application can be used to control the movement of the recording element of various types of electrodes, including 1) simple solid conductors, such as discs and needles (singles or arrays, often insulated except for the tip), 2) tracings on printed circuit boards, also insulated except for the tip, and 3) hollow tubes filled with an electrolyte, such as glass pipettes filled with potassium chloride solution or another electrolyte solution. The principal preparations include 1) living organisms, 2) excised tissue (acute or cultured), 3) dissociated cells from excised tissue (acute or cultured), 4) artificially grown cells or tissues, or 5) hybrids of the above.

If an electrode is small enough (micrometers) in diameter, then a skilled artisan may choose to insert the tip into a single cell, for example, a single neuron. Such a configuration allows direct observation and recording of the intracellular electrical activity of a single cell. However, at the same time such invasive setup may reduce the life of the cell and causes a leak of substances across the cell membrane. Intracellular activity may also be observed using a specially formed glass pipette, such as a hollow glass pipette, containing an electrolyte. In this technique, the microscopic pipette tip may be pressed against the cell membrane, to which it tightly adheres to by an interaction between the glass and the lipids of the cell membrane. The electrolyte within the pipette may be brought into fluid continuity with the cytoplasm by delivering a pulse of pressure to the electrolyte in order to rupture the small patch of membrane encircled by the pipette rim (whole-cell recording). Alternatively, ionic continuity may be established by "perforating" the patch by allowing exogenous pore-forming agent within the electrolyte to insert themselves into the membrane patch (perforated patch recording). Finally, the patch may be left intact (patch recording).

A skilled artisan may choose not to insert the tip into a single cell. Instead, the electrode tip may be left in continuity with the extracellular space. If the tip is sufficiently small, such a configuration may allow indirect observation and recording of action potentials from a single cell. Such process is termed single-unit recording. Depending on the preparation and precise placement, an extracellular configuration may pick up the activity of several nearby cells simultaneously, and this is termed multi-unit recording.

As electrode size increases, the resolving power decreases. Larger electrodes are sensitive only to the net activity of many cells, termed local field potentials. Still larger electrodes, such as un-insulated needles and surface electrodes used by clinical and surgical neurophysiologists, are sensitive only to certain types of synchronous activity within populations of cells numbering in the millions.

In addition to recording signals form cells, an electrode can also be used to deliver stimulus to cells, such as delivering electric stimulus (current stimulus or voltage stimulus), as well as chemical stimulus, including, but not limited to small molecule compounds, peptides, antibodies and other therapeutic agents.

The term "retraction" refers to the movement of the recoding tip (distal end) of the electrode towards the supporting tip (proximal end). In one embodiment, "retraction" of an electrode results in withdraw of the recording tip of an electrode from the targeted tissue or cell.

The term "elongation" refers to the movement of the recoding tip (distal end) of the electrode away from the supporting tip (proximal end). In a typical example, "elongation" of an electrode results in the penetrating of the recording tip of an electrode deeper into the targeted tissue or cell.

The term "bridging device" refers to a micro-bridge connecting two driving apparatuses of the present, for example, two shape memory alloy wires individually connected to a circuit and receiving commanding currents. The device is typically made of rigid or semi- rigid materials, such as ceramic, hard plastic, etc.

The term "an optimal position" refers to a position where an electrode is able to record the best or optimal quality of the signals for electric activities of cells/tissues as compared to surrounding positions.

### I. Driving Apparatus

In one embodiment of the invention, the present application provides a driving apparatus that is capable of controlling elongation and/or retraction of the recording element of an electrode connected with said apparatus within a tissue or a cell. In a preferred embodiment of the invention, the present driving apparatus provides a bi-directional moving mechanism, driving an electrode to move along two opposite directions (elongation and/or retraction) as desired. Such a mechanism offers significant flexibility in finding the best recording sites of the electrode.

The driving apparatus comprises a shape memory alloy (SMA) wire connected with an electric circuit, which is capable of generating commanding current to SMA wire. Typically, the circuit is composed of individual electronic components, such as resistors, transistors, capacitors, inductors, and diodes, connected by conductive wires or traces through which electrical current can flow. The two ends of the SMA wire are connected to conductive wires of the circuit, for example, are either glued by a conductive epoxy or soldered to a conductive wire with lower impedance than that of the SMA wire. A commanding electric current is then generated by the power source and the current may be applied to the SMA wire. The commanding electrical current is generated by either a DC current system or a PWM (pulse width module) current, and can be instantly turned on and off to set up the SMA wire and its connected electrode at a desired position. The electric current generates heat when passing through the SMA wires. By controlling the intensity, the duration and the frequency of the commanding current, a desired level of heat can be generated in the SMA wire and retract the SMA wire to the desired degree. In an exemplary embodiment, the commanding current heats the SMA wire to a temperature of at least 20, 30, 40, 50, 60, 70, 80, 90, 100, 150, 200 or 300 °C. Depending on the type of the SMA wires used in the driving apparatus and its ability of changing length/shape in reaction to change of the temperature of the wire, the intensity/duration of the commanding current is determined to achieve the desired temperature resulting in the optimal length change of the SMA wire. In a typical embodiment, the SMA wire, which is stretched more than at least 1, 2, 3, 4, 5, 6, 7, 8, 10, 12, 15, 17, 20, 25, 30, or 50% of its original length, can be restored to its original position when heated.

After the commanding current is turned off, the SMA wire will cool down and restore up to its original un-stretched shape and length. The total heating process is achieved within 1, 2, 3, 5, 7, 10, 20, 30, 40 seconds, or within 1, 3, 5, 10, 30 minutes. The heating process can also be divided into several cycles, for example, at least 2, 3, 4, 5, 7, 10, or 20 cycles during the heating process. The heat produced during the process is dissipated very quickly since the diameter of SMA wire used is very small, for example, less than 20, 50, 100, 200, 300, 500, 700 or 800 µm, or less than 1, 5, 10 mm. For a selected SMA, the range of length change is determined by the original length of SMA wire, which is at least 10, 100, 500, 1000µm, or 1, 3, 5, 10, 50, 100, 1000 mm.

The total range of length change of a SMA wire is determined by the length before being exposed to heat and the percentage of the retraction when heated. Thus, typically, the total length change of the SMA wires of the present invention is at least 0.1, 0.5, 0.8, 1, 2, 3, 5, 7, 8, 10, 15, 20, 25, 50, 75, 100, 200, 300, 500, or 800 µm, or 1, 3, 5, 10, 50, or 100 mm.

In one embodiment, the SMA wire is a straight wire. In another embodiment, the SMA wire can be a curved wire. In yet another embodiment, the SMA wire may have a straight portion and a curved portion. In some embodiment, the SMA wire is not in serpentine shape, resembling the letter "s", a sine wave or a snake.

Although passing an electric commanding current is typically used in the present application to heat the SMA wires, other approaches that generate a desired level of heat in the SMA wires may also be used to heat the SMA wires. For all the embodiments, a suitable amount of AC alternating current which has a frequency less than 500 MHz can also be used to heat and therefore make the SMA wire(s) retract.

Typically, an electrode comprises a lumen, an elongated body, a proximal end and a distal end, with the distal end as the recording element (recoding tip) for recoding the electric activity of a cell or a tissue and the proximal end as the supporting element (supporting tip) to anchor the electrode to a desired position.

In one aspect, the electrode may be covered by a guiding tube. In another aspect, a guiding tube is not necessary. The driving apparatus that makes the recording element of the electrode extrude or retract is coupled to the proximal end, the distal end or the body of the electrode or the body of guiding tube in a way that the length change of the SMA wire results in the movement of the electrode. In an exemplary embodiment, the driving apparatus is attached at the proximal end of the guide tubing or the electrode. In one embodiment, the SMA wire is attached to the electrode or the guiding tube by side-gluing into the electrode or the tube longitutively. In another embodiment, the wire can be coupled to the electrode or the guiding tube via a bridge that is capable of move the electrode when the wire retracts.

The commanding electrical current, once applied to the wire, retracts the wire, with a retracting distance depending on the intensity, frequency or duration of the commanding current as well as the physical characteristic and the length of the wire. This will provide the driving force to move the recording element of the electrode connected with the wire.

The driving ability of the apparatus may be accomplished via a shape memory alloy wire or a wire having similar length-changing characteristics as a shape memory wire. Many shape memory wires can be chosen to achieve this task as discussed in the "definition". In an exemplary embodiment, nitinol wires are selected for making the apparatus. As a shape memory alloy, the nitinol wire can be stretched up to about 108% of its original length. Upon applying heating to around 60 °C, the nitinol wire will recover up to its original length. This process will provide the driving force to move the recording tip of the electrode connected with the wire.

The diameter of the SMA wire can be very small, less than 500, 300, 200, 100, 80, 60, 50, 40, 30, 20 or 10 µm. In an exemplary embodiment of the present application, the diameter of the nitinol wire can be as small as several tens of microns, and the attached wire can be even thinner. In one embodiment, each driving unit (apparatus) can be made sufficiently small, such as less that 10, 50, 100, 200, 300, 400, 500, 800, or 1000 µm to fit in a compact array plan. In a preferred embodiment, the driving apparatus has a diameter less than 100, 200, 300, 400, 500, 600, 700, 800, 900 or 1000 µm. In a more preferred embodiment, the driving apparatus has a diameter less than 300 µm. The diameter of the driving apparatus is the diameter of the circle enclosing the electrode connected to the SMA wire and equals to the value of the diameter of the electrode plus the diameter of the SMA wire(s) and the thickness of the connecting layers.

In an exemplary embodiment, the driving apparatus has only one driving SMA wire with a diameter of less than about 50 µm connected to a microelectrode with a diameter of less than about 100 µm with a thin layer of glue with a thickness of less than 50 µm. The diameter of the driving apparatus is thereby less than 200 µm.

In another exemplary embodiment, the driving the driving apparatus has two driving SMA wire with a diameter of less than about 50 µm connected to a microelectrode with a diameter of less than about 100 µm with two thin layers of glue with a thickness of less than 50 µm. The diameter of the driving apparatus is thereby less than 300 µmIn addition to the adjustable mechanism described above, which is a single-use model with SMA wire pre-stretched, and shrinks upon the command (heat). The present device also includes a mechanism of restoring the stretched state of the wires and thus, the wires may be re-used more than once.

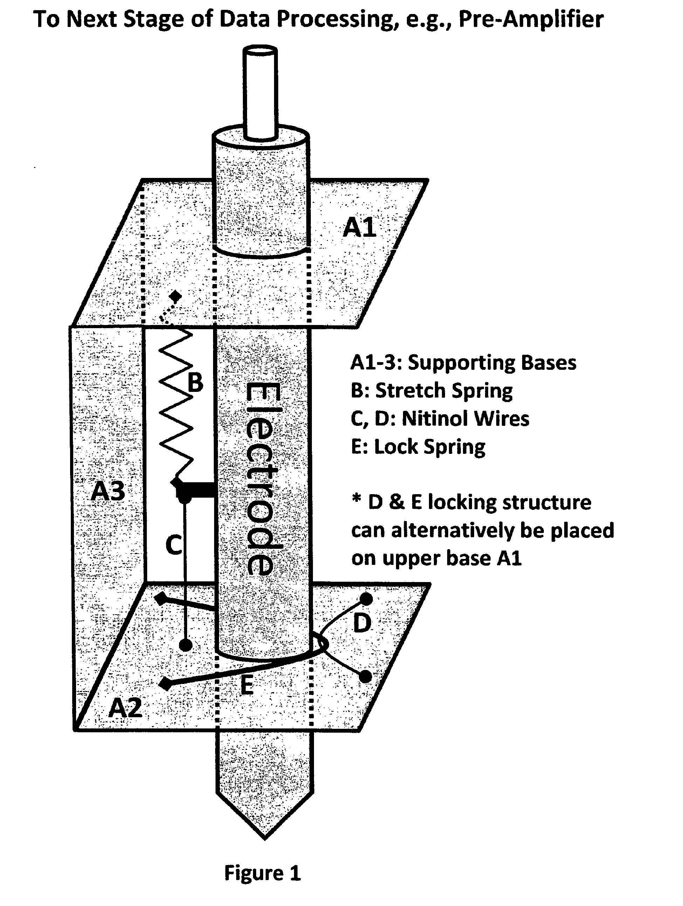

In a preferred embodiment of the invention, the retracted SMA wire is connected with a biasing element, such as a mini-spring, using means known in the art, for example by conductive glue, conductive epoxy or soldering, etc. As demonstrated in Figure 1, the SMA wire is connected to a mini-spring with a diameter of less than 100 µm, 500 µm, 1 mm, 5 mm, or 1cm,. After the wire is retracted due to the heat generated by a commanding current, the spring is strong enough to pull the SMA wire back to the stretched mode, and thus pull the electrode back to its original position.

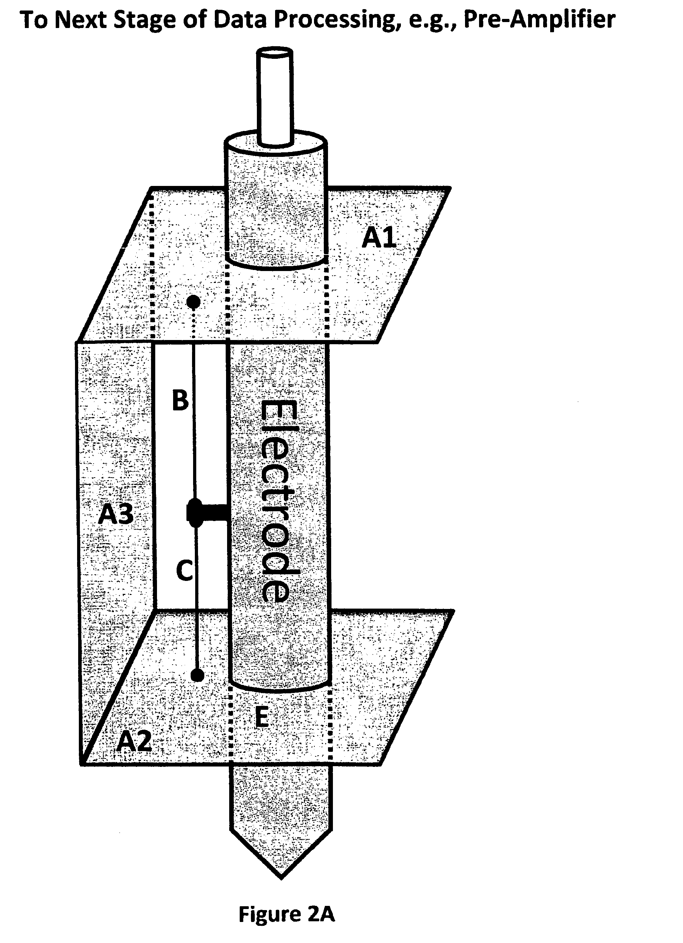

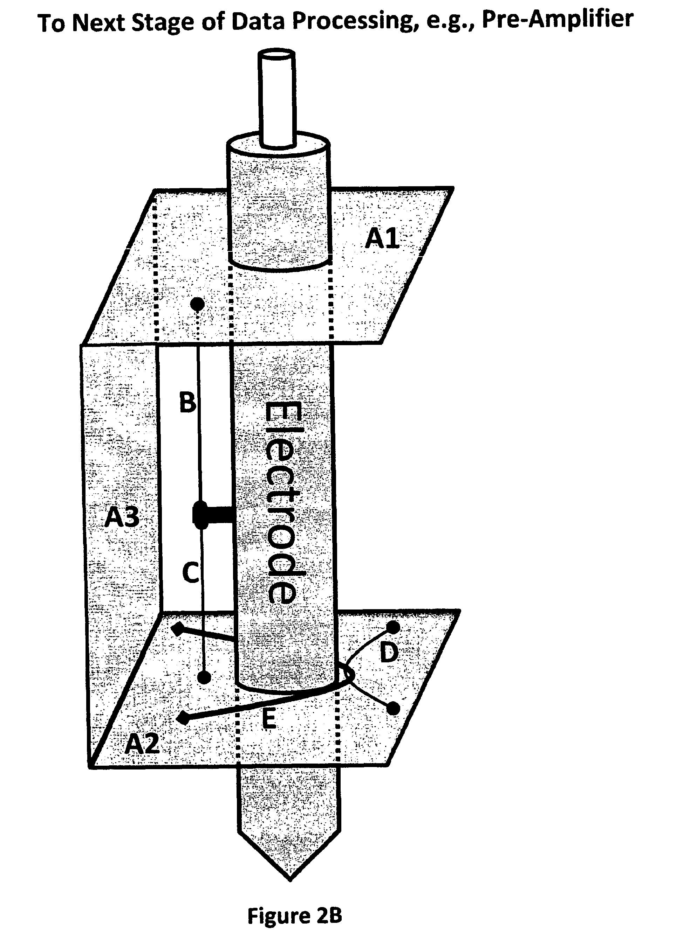

In examplary embodiment, the SMA wire is further connected with another SMA wire connected with a commanding circuit. The SMA wire can pull the electrode in the opposite direction when receives a commanding current. The dual SMA wire driving system is able to control the movement of the electrode in both directions. An exemplary design with the dual SMA wire driving apparatus is shown in Figure 2A and 2B. In particular, SMA wire B and C receive commanding currents independently. Retraction of B will move the electrode upward while retraction if C will move the electrode downward.

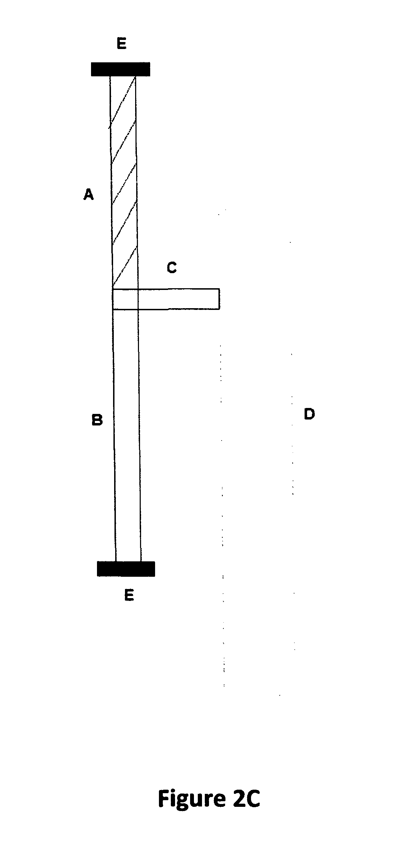

In yet another examplary embodiment, the driving SMA can be both one part of a single piece of nitinol wire. As shown in Figure 2C, when part A of the wire is in contracted mode, B is in elongated or stretched mode. Between A and B a common low impedance wire for applying electricity to one end of either A or B is also attached, it is not necessary to have two separate connecting point for A and B. The other end for applying current is at the bases of part A or part B. When there is current follow through either A or B due to voltage difference between the base and this common wire, the corresponding part is contracted. At the same time, the voltage difference of the other part are kept the same, so there is no current follow, therefore that part is stretched by the contracted part. As a result, the desired dual directional movement of the electrode is achieved via one SMW wire using the design shown in Figure 2C. The locking mechanism can be combined within this embodiment.

In some embodiments of the invention, a locking device is used together with the restoring mechanism described herein to move and lock the electrodes in any desired positions. Specifically, as shown in Figure 1 the SMA wire (C) controlling the insertion/retraction of electrode is constantly stretched by the vertical spring. Another SMA wire (D) is stretched by a horizontal spring load (E), forming a locking device. This horizontal load tightly locks the electrode when the electrode is not being adjusted. When the first round of retraction of the C SMA wire starts, a heat command is also applied to the D SMA wire, retraction of which loosens the lock of the electrode from the spring load. Consequently, the recording element of the electrode moves downward until arriving at a desired position. The heat command is then terminated for both the C and DSMA wires. As such, the electrode is locked again by the locking device (E) at this position. When the position of the electrode needs to be readjusted, e.g., retraction of the electrode is needed, only D SMA wire is heated, loosening the lock of the electrode. Under this condition, the C SMA wire, which remains un-heated, can be stretched back to its original position by the force of the spring. Apparently, all the operations require the spring load to be finely balanced with the strength of the SMA wire and the timing for heating different SMA wires to be precisely coordinated.

The locking device described herein can also be used together with the dual SMA wire driving systems described herewith as shown in Figure 2B.

The locking device can be located at any position along the electrode as long as retains its locking function, for example, can be on either side of the supporting plane as shown in Figure 1.

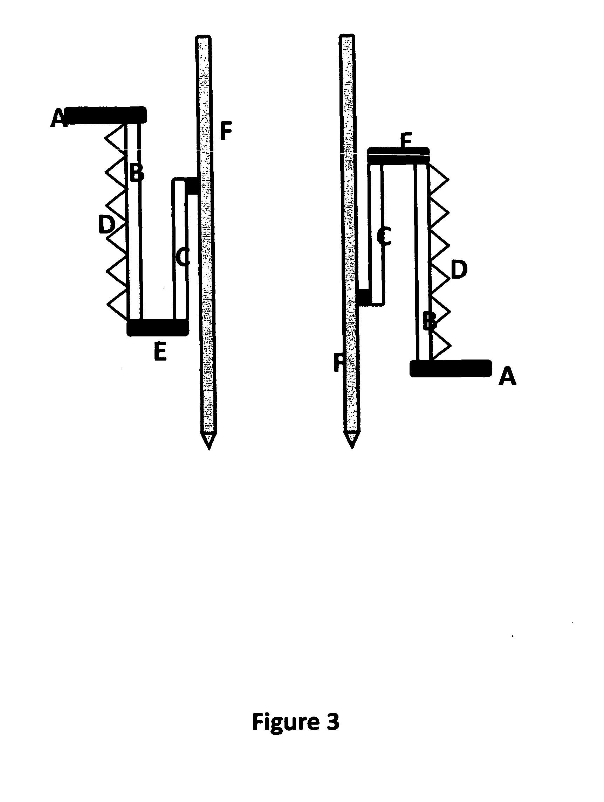

The retraction and elongating of the electrode can also be achieved via a dual-driving device comprising two driving apparatuses described herein as shown in Figure 3. In particular, such a device includes two micro-driving apparatuses, each of which comprises a shape memory alloy wire individually connected to a commanding circuit. The two SMA wires in each apparatus are in parallel position and are connected by a bridging device that is vertically coupled to both wires. In a exemplary embodiment, one wire is side-glued to the electrode with one end vertically connected with one end of the bridge while the other wire has one end connected to a fix point (such as a skull) and the other end vertically connected to the other end of the bridge. As a result, when giving the commanding current, the retraction of one SMA wire will move the electrode in one direction while, the retraction of the other SWA wire will move the electrode to the opposite direction. Thus, the movement of the electrode can be controlled in both directions (forwarding and withdrawing) by controlling which SMA wire is given a commanding current. The more detailed description of this dual-driving device is provided in Example 1.

### II. Adjustable Electrodes

The present application includes an adjustable electrode apparatus comprises electrode coupled to the driving apparatus described herein. In a preferred embodiment, the electrode apparatus further comprises the locking device described above.

In an exemplary embodiment, the electrode apparatus comprises: 1) an electrode comprising a lumen, an elongated body, a proximal end and a distal end, 2) a shape memory alloy wire coupled to the proximal end of the microelectrode for extending and retracting the electrode, wherein the alloy wire is stretched with a biasing element; and 3) a commanding circuit connected to the alloy wire for providing an electrical current to the wire.

Using the driving apparatus of the present application, a skilled artisan can freely elongate or retract, and lock an electrode t in a desired position. The electrode can be any biosensor, with the proximal end of said electrode is connected to a preamplifier, which is further connected to a data processing system (for example, preamplifier). The electrode is configured to measure an electrical physiological characteristic or signal of a cell and is adapted for contact with a cell within a tissue. The driving apparatus of the present invention can also be used together with multiple recording site electrodes to collect data from multiple sites when locked at any desired position.

The driving apparatus of the present invention can be used to control the retraction and elongation of various types of electrodes available in the art as discussed in the "definition", which are used for a variety of recording purposes in the field of electrophysiology.

In one aspect, the electrodes of the present application are suitable for intracellular recording, which involves measuring voltage and/or current across the membrane of a single cell. To make an intracellular recording, the tip of a fine (sharp) microelectrode must be inserted inside the cell, so that the membrane potential can be measured. Typically, the resting membrane potential of a healthy cell will be -60 to -80 mV, and during an action potential the membrane potential might reach +40 mV. Most microelectrodes used for intracellular recording are glass micropipettes, with a tip diameter of less than 1 µm, and a resistance of several megaOhms. The micropipettes are filled with a solution that has a similar ionic composition to the intracellular fluid of the cell. A chlorided silver wire inserted in to the pipette connects the electrolyte electrically to the amplifier and signal processing circuit. The voltage measured by the electrode is compared to the voltage of a reference electrode, usually a silver chloride-coated silver wire in contact with the extracellular fluid around the cell. In general, the smaller the electrode tip, the higher its electrical resistance, so an electrode is a compromise between size (small enough to penetrate a single cell with minimum damage to the cell) and resistance (low enough so that small neuronal signals can be discerned from thermal noise in the electrode tip).

In one aspect of the present application, the electrodes are used in the recording with "voltage clamp" techniques. The voltage clamp technique allows an experimenter to "clamp" the cell potential at a chosen value. This makes it possible to measure how much ionic current crosses a cell's membrane at any given voltage. This is important because many of the ion channels in the membrane of a neuron are voltage gated ion channels, which open only when the membrane voltage is within a certain range. Voltage clamp measurements of current are made possible by the near-simultaneous digital subtraction of transient capacitive currents that pass as the recording electrode and cell membrane are charged to alter the cell's potential.

In another aspect of the present application, the electrodes are used in the recording with "current clamp" techniques. The current clamp technique records the membrane potential by injecting current into a cell through the recording electrode. Unlike in the voltage clamp mode, where the membrane potential is held at a level determined by the experimenter, in "current clamp" mode the membrane potential is free to vary, and the amplifier records whatever voltage the cell generates on its own or as a result of stimulation. This technique is used to study how a cell responds when electric current enters a cell; this is important for instance, for understanding how neurons respond to neurotransmitters that act by opening membrane ion channels. Most current-clamp amplifiers provide little or no amplification of the voltage changes recorded from the cell. The "amplifier" is actually an electrometer, sometimes referred to as a "unity gain amplifier"; its main job is to change the nature of small signals (in the mV range) produced by cells so that they can be accurately recorded by low-impedance electronics. The amplifier increases the current behind the signal while decreasing the resistance over which that current passes. The electrometer changes this "high impedance signal" to a "low impedance signal" by using a voltage follower circuit. A voltage follower reads the voltage on the input (caused by a small current through a big resistor). It then instructs a parallel circuit that has a large current source behind it (the electrical mains) and adjusts the resistance of that parallel circuit to give the same output voltage, but across a lower resistance.

In yet another aspect, the electrodes of the present application are used in the "patch clamp" technique. This technique was developed by Erwin Neher and Bert Sakmann who received the Nobel Prize in 1991. Conventional intracellular recording involves impaling a cell with a fine electrode; patch-clamp recording takes a different approach. A patch-clamp microelectrode is a micropipette with a relatively large tip diameter. The microelectrode is placed next to a cell, and gentle suction is applied through the microelectrode to draw a piece of the cell membrane (the 'patch') into the microelectrode tip; the glass tip forms a high resistance 'seal' with the cell membrane. This configuration is the "cell-attached" mode, and it can be used for studying the activity of the ion channels that are present in the patch of membrane. If more suction is applied, the small patch of membrane in the electrode tip can be displaced, leaving the electrode sealed to the rest of the cell. This "whole-cell" mode allows very stable intracellular recording. A disadvantage (compared to conventional intracellular recording with sharp electrodes) is that the intracellular fluid of the cell mixes with the solution inside the recording electrode, and so some important components of the intracellular fluid can be diluted. A variant of this technique, the "perforated patch" technique, tries to minimize these problems. Instead of applying suction to displace the membrane patch from the electrode tip, it is also possible to make small holes on the patch with pore-forming agents so that large molecules such as proteins can stay inside the cell and ions can pass through the holes freely. Also the patch of membrane can be pulled away from the rest of the cell. This approach enables the membrane properties of the patch to be analyzed pharmacologically.

In situations where one wants to record the potential inside the cell membrane with minimal effect on the ionic constitution of the intracellular fluid, a sharp electrode can be used (sharp electrode technique). These micropipets (electrodes) are again like those for patch clamp pulled from glass capillaries, but the pore is much smaller so that there is very little ion exchange between the intracellular fluid and the electrolyte in the pipette. The resistance of the electrode is in 10s or 100s of MΩ in this case. Often the tip of the electrode is filled with various kinds of dyes, such as Lucifer yellow, to fill the cells recorded from, for later confirmation of their morphology under a microscope. The dyes are injected by applying a positive or negative, DC or pulsed voltage to the electrodes depending on the polarity of the dye.

In another aspect, the present application provides electrodes used in extracellular recording of a cell. In one embodiment, the electrodes of the present application are used in "single-unit recording". In particular, an electrode introduced into the brain of a living animal will detect electrical activity that is generated by the neurons adjacent to the electrode tip. If the electrode is a microelectrode, with a tip size of about 1 µm, the electrode will usually detect the activity of at most one neuron. Recording in this way is generally called "single-unit" recording. The action potentials recorded are very like the action potentials that are recorded intra-cellularly, but the signals are very much smaller (typically about 1 mV). Most recordings of the activity of single neurons in anesthetized animals are made in this way, and all recordings of single neurons in conscious animals. Recordings of single neurons in living animals have provided important insights into how the brain processes information. For example, David Hubel and Torsten Wiesel recorded the activity of single neurons in the primary visual cortex of the anesthetized cat, and showed how single neurons in this area respond to very specific features of a visual stimulus.. J Physiol. 148:574-91 (1959); J Physiol. 160:106-54 (1962) Hubel and Wiesel were awarded the Nobel Prize in Physiology or Medicine in 1981.If the electrode tip is slightly larger, then the electrode might record the activity generated by several neurons. This type of recording is often called "multi-unit recording", and is often used in conscious animals to record changes in the activity in a discrete brain area during normal activity. Recordings from one or more such electrodes which are closely spaced can be used to identify the number of cells around it as well as which of the spikes come from which cell. This process is called spike sorting and is suitable in areas where there are identified types of cells with well defined spike characteristics. If the electrode tip is bigger still, generally the activity of individual neurons cannot be distinguished but the electrode will still be able to record a field potential generated by the activity of many cells.

In an exemplary embodiment, the electrode of the present invention is made of fused silica tubing and the insulator, for example, coated with polyimide, or, alternatively without coating. The inner diameter (ID) of tubing ranges from 1 µm to 1 mm, preferably, from several to a couple of hundred µm, more preferably, from 10-100 µm. In one embodiment, there is a tungsten wire placed inside the tubing. In an alternative embodiment, the tubing can be filled with high concentration of electrolyte with the ends sealed. In one aspect, the seal may be made with conductive epoxy or glue. In a preferred embodiment, the electrolytes is potassium chloride or other salts at a concentration of from 0.1 to 10M, preferably from 1-7M, and more preferably, from 3-5M. One end of the electrode is placed within the brain tissue for signal collection, whereas the other end is connected to the preamplifier for data acquisition and amplification. In one embodiment, the electrode is longer than the guide tubing by certain length (typically -10 µm) at a pre-set condition. This electrode tubing is within a guide tubing fused with silica or PEEK (Polyetheretherketone) tubing, which has high biocompatibility and rigidity. The outer diameter (OD) of the fused silica tubing is 2-5 µm less than that of the ID of guide tubing to minimize the friction when sliding. All guide tubings are bundled to form the frame of recording array (see Fig. 2). The distance between the guide tubing, the overall shape/contour of the array, and the length of each tubing can all be pre-adjusted to meet special recording need.

In another aspect, the electrodes of the present application can be used for recording the extracellular filed potentials. The extracellular field potentials are the electrical potential produced by cells, e.g. nerve or muscle cells, outside of the cell. They are local current sinks or sources that are generated by the collective activity of many cells. Electrophysiological studies investigate these potentials using extracellular microelectrodes. Usually a field potential is generated by the simultaneous activation of many neurons by synaptic transmission. A signal is recorded using a low impedance extracellular microelectrode, placed sufficiently far from individual local neurons to prevent any particular cell from dominating the electrophysiological signal. This signal is then low-pass filtered, cut off at ~300 Hz, to obtain the local field potential (LFP). The low impedance and positioning of the electrode allows the activity of a large number of neurons to contribute to the signal. The unfiltered signal reflects the sum of action potentials from cells within approximately 50-350 µm from the tip of the electrode (Legatt 1980; Gray 1995)^{[1][2]} and slower ionic events from within 0.5-3 mm from the tip of the electrode (Juergens 1999). The low-pass filter removes the spike component of the signal and passes the lower frequency signal, the LFP.

The voltmeter or analog-to-digital converter to which the microelectrode is connected measures the electrical potential difference (measured in volts) between the microelectrode and a reference electrode. One end of the reference electrode is also connected to the voltmeter while the other end is placed in a medium which is continuous with, and compositionally identical to the extracellular medium. In a simple fluid, with no biological component present, there would be slight fluctuations in the measured potential difference around an equilibrium point, this is known as the thermal noise. This is due to the random movement of ions in the medium and electrons in the electrode. However, in neural tissue the opening of an ion channel results in the net flow of ions into the cell from the extracellular medium, or out of the cell into the extracellular medium. These local currents result in larger changes in the electrical potential between the local extracellular medium and the interior of the recording electrode. The overall recorded signal thus represents the potential caused by the sum of all local currents on the surface of the electrode.

In yet another aspect, the electrodes of the present application can be used for in planar patch clamp. Planar patch clamp is a novel method developed for high throughput electrophysiology. Instead of positioning a pipette on an adherent cell, cell suspension is pipeted on a chip containing a microstructured aperture. A single cell is then positioned on the hole by suction and a tight connection (Gigaseal) is formed. The planar geometry offers a variety of advantages compared to the classical experiment: - it allows for integration of microfluidics, which enables automatic compound application for ion channel screening; - the system is accessible for optical or scanning probe techniques ; perfusion of the intracellular side can be performed.

### III. Adjustable Multi-electrode System (AMES)

In one aspect, the present application includes an adjustable multiple electrode system (AMES) comprising one or more arrays of the electrodes of the present invention, each of which can be individually adjusted via the driving apparatus disclosed herein.

The AMES comprises three major components to achieve the adjustable property: 1) the driving apparatus of the present invention controlling elongation and retraction of the recording element of an electrode; 2) the locking device disclosed herein to lock the electrode to a desired position; and 3) individualized control of each electrode composed by AMES. These three components are interwoven in the system as formulated in the diagram (Figure 1).

The basic design of AMES is as follows: multiple electrodes are pre-aligned within a cannula, which is made by standard biocompatible materials, including, but not limited to, plastic, polyetheretherketone, alloy, or stainless steel. At the top end of the cannula, the electrodes are individually connected to the preamplifier, which further transfer/connect to the data processing system. Each electrode is connected with a driving apparatus described herein, which controls the elongation or withdrawal of the electrode upon command. Each electrode can be elongated up to 5000 µm, preferably, 1000 µm, and more preferably, 400 µm. This work zone enables the electrode to penetrate through a distance crossing more than, 5, 10, 20, 30, 50, 100 or 200 cells, for example, neurons. Thus, there are more than 10, 20, 30, 50, 100, 200 or 500 opportunities for each electrode to obtain neuronal signals. In the middle of each electrode, there is a locking device discussed herein wrapping around the electrode and lock the electrode once the recording tip of the electrode reaches its desired position. When many of these individually drivable electrodes are bundled together, an array of AMES is formed. The number of electrodes, the shape and contour of the array can be adapted to meet different research requirements. Typically, the AMES of the present applications contains at least 2, 5, 10, 50, 100 or 500 arrays, preferably, contains 5-100 arrays, and more preferably, contains 5-50 arrays. The AMES may contain at least 2, 5, 10, 50, 100, 500, 1000, 5000 or 10,000 electrodes, preferably contain 2-1000 electrodes, and more preferably contains 10-500 electrodes.

The electrodes are pre-lined in several optional arrays, each of which provides different tracks for electrodes (see Figure 3). As such, the direction of each electrode can be pre-set to form various shapes and contours of AMES, which should be fit well into the shape of the targeted tissues, nuclei or cells. In an exemplary embodiment of the present application, the electrodes are arranged in a way to radiate to a spherical surrounding with different lengths so that the AMES has a shape and contour fitting into a sphere. As a result, a sphere or other 3D shapes of tissues can be sampled for recording. This function is particularly useful to examine the neural activity of a whole nucleus in the brain with the activity of individual neurons within the whole nucleus being recorded simultaneously. The AMES of the present application allows the location of each electrode tip within the brain tissue to be determined using software-based analysis. In particular, the position of the tip of each electrode in the array plat can be determined based on the original position of each electrode and the degree of protrusion. The software can then calculate the relative positions of all electrode tips to form a network image in the computer.

In another aspect, the present application takes advantage of an important feature of SMA wire to condense a large number of recording electrodes in a small array template. First, because the diameter of the SMA wire can be as small as several tens of microns, and the attached wire can be even thinner, each driving unit can be made sufficiently small (for example, less than 2000, 1000, 500, 300, 200 or 100 microns) to fit in a compact array plan. Second, by assigning the driving units at different plans, more space is generated and more electrodes can be arrayed within a multi-channel unit. With this design, an array plate of 2 cm by 2 cm can accommodate at least 40, 60, 100, 200, 300, 400, 500, 700, 1000 or 5000 electrodes. Third, probably the most striking feature of AMES offers is that electrodes within the AMES can be adjusted individually in the behaving animal to obtain the optimal recording signal, because the command transmission and electrode adjustment are both achieved without direct contact with the animal. Fourth, the design of fused silica tubing minimizes the size of the guide cannula. As such, the related surgery trauma is minimized.

When recording the neuronal activities of a nucleus in a brain of the tested subject, in an exemplary embodiment, AMES is installed in a brain in a tested subject. Once installed into the brain, AMES can be individually adjusted within a range of 0-10, 0-20, 0-50, 0-100, 0-200, 0 - 400, 0-500, 0-600, 0-1000 or 0-5000 µm such that each electrode can be independently protruded until it reaches an optimal position to acquire neuronal signals. The electrode can then be locked at the selected position for the rest of the experiment (at least 1, 2, 5, 8, 10, 20, 50, 100, 200, 500 or 1000 hours to 1, 2, 5, 8, 10, 20, 30 or 50 weeks) or can be re-adjusted upon further experimental needs. Thus, every electrode within the multi-electrode system is able to acquire valid signals. Signals outputted by the electrode array are fed to a device or devices that are carried by the experimental animal(s). The processed signals can then be sent wirely or wirelessly to the next stage of data processing. The adjustment of electrodes can be achieved by a computer-software through wireless signals. The AMES allows recording electric activities of a large number of neurons simultaneously such that the activities of a neural circuitry comprising these neurons can be formulated and assessed. Subsequently, the AMES provides a powerful tool in exploring the real time neural activity associated with specific behavioral outputs. Taken together, this adjustable multi-electrode system substantially improves the efficacy of signal acquisition and offers an unprecedented power for in vivo recordings.

Another feature of the AMES is that each individual electrode can be used as a stimulation electrode, delivering current or voltage stimulus to the positioned site. To do this, the current output is by-passed through the preamplifier, and is directly applied to the tip of the electrode. Moreover, different electrodes can deliver the same or different patterns of stimuli at the same or different time. At the same time, electrodes that are not used as stimulators can still function as recording electrodes, collecting signals. Thus, the input and output neurons can be examined at the same time within a small neural circuit. In addition to electric stimulus, the electrode of the AMES can also deliver chemical stimulus, such as small molecule compounds, peptides, polypeptides, antibodies, and other candidate therapeutic agents. The recording electrodes can monitor the changes in electric activity of neurons in response to these chemical stimuli.

A variation of AMES electrode is that one electrode can be made with multiple recording sites; these recording sites are connected to a single multiplexer. The output of the multiplexer is, in turn, connected to the preamplifier. During recording, signals from multiple sites can be simultaneously monitored in real time; signals from these multiple sites can be all recorded or selectively recorded, depending on the actual experimental need.

The AMES of the present application is also useful in recording electric activities of a cell, a group of cells or a tissue derived from various subjects, including both invertebrate (for example, insects, sea slugs, etc.) and vertebrates (for example, fishes, frogs, rodents (rabbits, mice or rats, etc), cats, dogs, and monkeys) and human. In a preferred embodiment, the cell is a neuron or a muscle cell and the group of cells is a nucleus of a brain or a ganglion of a nervous system.

In another aspect, the AMES is suitable to record neuronal activities *in vivo* of a nucleus in a freely-moving animal, as well as, in the anaesthetized animals or in vitro/ex vivo conditions (e.g., brain slices). Indeed, for in vitro studies involving multi-electrodes, a common technical issue is the difficulty of placing multiple micromanipulators within a limited working space. The design of AMES largely solves this particular problem.

The present AMES is extremely useful in at least the following two special circumstances. First, recordings aim to understand the working of micro-circuitry require simultaneously obtaining signals from a large number of neurons. The adjustable property increases the number of "working" electrodes and thus effectively increases sample size within the micro-circuitry. Furthermore, the relative positions of all electrodes are simultaneously obtained. This additional information is particularly useful when the summation or integration of neuronal activities is required for computations involving consideration of spatial or relaying effects. Second, recordings aims to monitor neuronal activities over a prolonged period of time are not always successful using the traditional multi-electrode system; the signals may be lost during the prolonged period due to slightly draft of the electrode from the target neurons. Using the present AMES, neuronal activities from the presumed same neuron can be traced and controlled at the maximal acquisition level by adjusting the position of the electrode during the recording period. Thus, the high failure rate for chronic in vivo recording can be effectively prevented. Thus, with the present AMES, the difficult multi-unit recordings can be performed in regular laboratories.

The foregoing written specification is considered to be sufficient to enable one skilled in the art to practice the invention. Indeed, various modifications of the embodiments in addition to those shown and described herein will become apparent to those skilled in the art from the foregoing description and fall within the scope of the appended claims.

It is understood that the application of the teachings of the present invention to a specific problem or situation will be within the capabilities of one having ordinary skill in the art in light of the teachings contained herein.

Further details of the invention are illustrated by the following non-limiting Examples. The disclosures of all citations in the specification are expressly incorporated herein by reference.

### EXAMPLES

### Example 1

This example illustrates the installation of the AMES in the rodent brain. As shown in Figure 3, in which A is a fixed point (normally the upper skull), B and C are two nitinol wires that are pre-stretched and retract upon commands through the commanding circuits (described above). The lower end of B is attached to A. B and C are harnessed together by E, which is a micro-bridge made of rigid materials (e.g., ceramic, hard plastic). D is a micro-spring or tubing with the same length with a balanced strength that maintains the extended shape of B. The other end of C is attached to F, the recording component. When C retracts upon the controlled circuit (via heating), it pulls F to protrude from the guide tubing to the deeper part of the tissue. The electrical activity is on-line monitored and simultaneously recorded through F, together with the relative position of F. To return to F's pre-set location, heat generated by current applied to B will make the E being lifted, therefore the F will withdraw from its maximum extruded location to the optimal (or near optimal) location. D is a microspring which keeps the nitinol wire B straight thus when the wire shortens its length, the force generated will be fully applied on the electrode. This micro-spring can be eliminated if careful arrangement to make nitinol wire B straight. This is the basic design, based on which variations can be developed. For example, in the above set up, B can be retracted (via heat control) first to pull the F into the deeper tissue; the retraction of F can be achieved by retraction of C (via the heat-based control described above).

## Claims

1. An adjustable electrode apparatus comprising
an electrode comprising a lumen, an elongated body, a proximal end and a distal end, a shape memory alloy wire (C) coupled to the proximal end of the microelectrode for extending and retracting the microelectrode, wherein the alloy wire is adapted to be stretched with a biasing element (B); and
a commanding circuit connected to the alloy wire for providing an electrical current to the wire;
**characterized in that** said apparatus further comprises a locking device (D, E) to lock the electrode in a desired position.

2. The apparatus of Claim 1, wherein said locking device (D, E) locks the electrode in a desired position via a spring (E) horizontally wrapping the electrode.

3. The apparatus of Claim 2, wherein a curved shape memory alloy wire (D) is stretched by said spring (E) wrapping the electrode,
wherein said spring (E) is positioned horizontally with said curved wire (D),
wherein said curved wire (D) connected to an electric circuit,
wherein said curved wire (D) is retracted when a commanding current is applied to the circuit.

4. The apparatus of Claim 3, wherein the retraction results in loosening lock of the electrode by said locking device (D, E) so that the position of the electrode can be readjusted.

5. The apparatus of Claim 1, wherein said electrode is adapted to be elongated or retracted by said driving apparatus through a group of neurons.

6. An adjustable multi-electrode system comprising at least one array of electrode apparatus of any one of Claims 1-5, wherein the position of each electrode of said system is individually adjustable.

7. The multi-electrode system of Claim 6, wherein said electrodes are adapted to measure neuronal signal of an awake animal.

## Patentansprüche

1. Eine einstellbare Elektrodenvorrichtung umfassend
eine Elektrode umfassend ein Lumen, einen länglichen Körper, ein proximales und ein distales Ende, ein mit dem proximalen Ende der Mikroelektrode zum Ausfahren und Zurückziehen der Mikroelektrode verbundenes Formgedächtnislegierungskabel (C), wobei das Legierungskabel angepasst ist, mit einem Beeinflussungselement (B) gedehnt zu werden, und
einen mit dem Legierungskabel verbundenen Steuerungskreislauf, um dem Kabel einen elektrischen Strom bereitzustellen;
**dadurch gekennzeichnet, dass** die Vorrichtung weiter eine Feststellvorrichtung (D, E) umfasst, um die Elektrode in einer gewünschten Position festzustellen.

2. Die Vorrichtung von Anspruch 1, wobei die Feststellvorrichtung (D, E) die Elektrode durch eine die Elektrode horizontal umhüllende Feder (E) in einer gewünschten Position feststellt.

3. Die Vorrichtung des Anspruchs 2, wobei ein gebogenes Formgedächtnislegierungskabel (D) durch die die Elektrode umhüllende Feder (E) gedehnt wird,
wobei die Feder (E) mit dem gebogenen Kabel (D) horizontal positioniert ist,
wobei das gebogene Kabel (D) verbunden zu einem elektrischen Kreislauf ist,
wobei das gebogene Kabel (D) zurückgezogen wird, wenn ein Steuerungsstrom an den Kreislauf angelegt wird.

4. Die Vorrichtung von Anspruch 3, wobei das Zurückziehen im Lockern des Feststellens der Elektrode durch die Feststellvorrichtung (D, E) resultiert, so dass die Position der Elektrode nachgestellt werden kann.

5. Die Vorrichtung von Anspruch 1, wobei die Elektrode angepasst ist, durch die Antriebsvorrichtung durch eine Gruppe von Neuronen verlängert oder zurückgezogen zu werden.

6. Ein einstellbares Multi-Elektrodensystem umfassend wenigstens eine Anordnung der Elektrodenvorrichtung irgendeines der Ansprüche 1-5, wobei die Position jeder Elektrode des Systems individuell einstellbar ist.

7. Das Multi-Elektrodensystem von Anspruch 6, wobei die Elektroden angepasst sind, ein neuronales Signal eines wachen Tieres zu messen.

## Revendications

1. Appareil à électrode ajustable comprenant
une électrode comprenant un lumen, un corps allongé, une extrémité proximale et une extrémité distale, un fil en alliage à mémoire de forme (C) couplé à l'extrémité proximale de la microélectrode pour étendre et rétracter la microélectrode, dans lequel le fil en alliage est apte à être étiré avec un élément de sollicitation (B) ; et
un circuit de commande relié au fil en alliage pour fournir un courant électrique au fil ;
**caractérisé en ce que** ledit appareil comprend en outre un dispositif de blocage (D, E) pour bloquer l'électrode dans une position souhaitée.

2. Appareil selon la revendication 1, dans lequel ledit dispositif de blocage (D, E) bloque l'électrode dans une position souhaitée au moyen d'un ressort (E) enroulé horizontalement autour de l'électrode.

3. Appareil selon la revendication 2, dans lequel un fil en alliage à mémoire de forme incurvé (D) est étiré par ledit ressort (E) enroulé autour de l'électrode,
dans lequel ledit ressort (E) est positionné horizontalement par rapport audit fil incurvé (D),
dans lequel ledit fil incurvé (D) est relié à un circuit électrique,
dans lequel ledit fil incurvé (D) est rétracté lorsqu'un courant de commande est appliqué au circuit.

4. Appareil selon la revendication 3, dans lequel la rétraction entraîne un relâchement du blocage de l'électrode par ledit dispositif de blocage (D, E) afin que la position de l'électrode puisse être réajustée.

5. Appareil selon la revendication 1, dans lequel ladite électrode est propre à être allongée ou rétractée par ledit appareil d'entraînement par le biais d'un groupe de neurones.

6. Système multi-électrodes ajustable comprenant au moins un réseau d'appareils à électrode selon l'une quelconque des revendications 1 à 5, dans lequel la position de chaque électrode dudit système est ajustable individuellement.

7. Système multi-électrodes selon la revendication 6, dans lequel lesdites électrodes sont propres à mesurer un signal neuronal d'un animal éveillé.
